# EUROPEAN PATENT APPLICATION

(11) **EP 2 011 782 A1**
(43) Date of publication of application: **07.01.2009**
(21) Application number: 07111544.8
(22) Date of filing: 02.07.2007
(51) Int. Cl.: C07C 269/04, C07C 263/04

(54) **Process for the synthesis of carbamates using co2**

(71) Applicant: HUNTSMAN INTERNATIONAL LLC, Salt Lake City, Utah 84108 (US)
(72) Inventor: The designation of the inventor has not yet been filed
(74) Representative: Swinnen, Anne-Marie

(57) **Abstract**

Process for the synthesis of carbamate derivatives from amine compounds, alcohols and CO₂ in the presence of ionic liquids and optionally in the presence of a base.

## Description

### Field of the invention

This invention relates to a process for the preparation of carbamates using CO₂.

The carbamates in turn can be decomposed into isocyanates.

### Background of the invention

Isocyanates are key intermediates for the manufacture of polyurethanes. They are conventionally produced by the phosgenation of primary amines which results in the production of two equivalents of HCl as a by-product. Considering the environmental hazard and corrosion problems in the phosgenation it is important to develop a route without using phosgene. Synthesis based on carbon dioxide (CO₂) as C₁- source would provide a sustainable, green alternative, with a reduced variable cost compared to CO and Cl₂ and avoidance of the production of HCl as by-product.

Chem. Commun. (2001) p. 2238-2239 (M. Abla, J-C. Choi and T. Sakakura) discloses a one-pot synthesis of carbamates, using aliphatic amines, CO₂ under very high pressure (300 bar), tin complexes as catalyst, alcohol and an equimolar amount of a chemical drying agent such as acetals as a co-reagent resulting in by-product formation. The co-reagent requires extensive recycle and to obtain good selectivity, very high pressure is required (300 bar and more).

J. Org. Chem., 1995, 60, p.2820-2830 (W. McGhee, D. Riley, K. Christ, Y. Pan and B. Parnas) describes the reaction of amines with equimolar amounts of base, CO₂ and an excess of an alkylating agent resulting in carbamates and large amount of salts as by-product. These salts cannot be recycled making this option very expensive and generating a very large waste stream.

WO 95/18098 (McGhee et al.) relates to a process in which an amine is combined with at least two equimolar amounts of base, CO₂ and at least an equimolar amount of a chemically dehydrating agent resulting directly in isocyanates and large amounts of very difficult or non recyclable salts. At best, the dehydrating agent can be recycled at the expense of a multistep process, requiring strong bases and acids hereby producing other waste salt streams.

Recent research, Angew. Chem. Int. Ed. (2003, 42, p.3257-3260 (F. Shi, Y. Deng, T. SiMa, J. Peng, Y. Gu and B. Qiao)), has resulted in the synthesis of ureas from amines with high pressure CO₂ in the presence of a base and an ionic liquid (IL). Aromatic amines react very slowly and observed yields are low. Urea can be separated as a solid after addition of water. The base/IL system could be recovered and reused after water removal. Furthermore, it was stated that urea can easily be converted into carbamates in a separate step such that the carbamate can be decomposed into isocyanates. However, this is a multistep process requiring a lot of very complicated recycling. Transformation of urea with alcohol into carbamate will result in an equimolar amount of amine which needs to be separated from the carbamate and recycled to the urea making step.

Int. J. Mol. Sci. (2006), 7, p. 438-450 (S-I. Fujita, H. Kanamaru, H. Senboku and M. Arai), describes the preparation of aliphatic cyclic urethanes from amino alcohols and high pressure carbon dioxide using ionic liquid catalysts with alkali metal promoters and alcohol solvent. This article demonstrates the principle of making cyclic urethanes. Yield of the desired cyclic urethane is low and the ionic liquid is used to tune the selectivity of the reaction and has no influence on the total conversion of the amino alcohols. This suggests that it will be very difficult to make linear urethanes by intermolecular reactions and that the use of ionic liquids will not help to convert amines, CO₂ and alcohols effectively to urethanes.

An improved process for the synthesis of carbamate derivatives has now been found by reacting amine compounds, alcohols and CO₂ in the presence of ionic liquids and optionally a base and a co-solvent.

This route provides a clean one step process to make carbamates avoiding the need of co-reagents, formation of by-products and salt formation. It provides a non-phosgene method for the preparation of carbamates and the derived isocyanates by using CO₂ as a C₁ feedstock.

### Summary of the Invention

The reaction works in principle for any aliphatic and aromatic amine, any type of alcohol and ionic liquid, and any type of base can be used as a promoter.

Any aliphatic or cycloaliphatic ar arylaliphatic amine or polyamine may be employed in the present invention. Examples are methylamine, ethylamine, n-propylamine, isopropylamine, n-butylamine, isobutylamine, hexylamine, cyclopropylamine, cyclobutylamine, cyclohexylamine, laurylamine, stearylamine, 1,4-butylene diamine, 1,6-hexylene diamine, 1,5-naphthylene diamine, 1,4-cyclohexene diamine, 4,4'-dicyclohexylmethane diamine, 1,4-xylylene diamine, isophorone diamine, 2,2,4-trimethylhexamethylene diamine, metaxylene diamine, tetramethyl metaxylene diamine, 1,3-bis(aminomethyl)cyclohexane.

Examples of mononuclear aromatic monoamines which can be used include aniline, o-and m-substituted anilines such as toluidines and alkyl anilines, chloroanilines, fluoroanilines, anisidines and nitroanilines.

Examples of diamines or higher functionality amines which can be used include the diaminobenzene isomers such as 1,3-phenylene diamine, alkyl-substituted diaminobenzenes such as the isomers oftolulene diamine (TDA), isomers of methylene diphenylene diamine (MDA) such as 4,4'-MDA and mixtures of various isomers and homologues of aniline-formaldehyde condensates known generally as PMDA. Particularly suitable mixtures are those containing from about 65 per cent to about 80 per cent 2,4-tolulene diamine and the balance 2,6-tolulene diamine. The commercially available mixture containing about 80 per cent 2,4- and about 20 per cent 2,6-tolulene diamine is very useful.

Preferred aliphatic amines for use in the present invention include 1,6 hexylene diamine, 1,4-cyclohexane diamine, 4,4'-dicyclohexylmethane diamine, metaxylene diamine, isophorone diamine, tetramethyl metaxylene diamine and 1,3-bis(aminomethyl)cyclohexane.

Preferred aromatic amines for use in the present invention include isomers of methylene diphenylene diamine such as 4,4'-MDA and mixtures of various isomers and homologues of aniline-formaldehyde condensates known generally as PMDA and isomers of TDA.

The type of alcohol used is not critical.

Aliphatic, cycloaliphatic, arylaliphatic, aliphatic cycloaliphatic, aromatic, halogenated and any type of functionalised alcohol can be used. Examples are methanol, ethanol, n-propanol, isopropanol, butanol, pentanol, hexanol, 3,3-dimethyl-2-butanol, cyclohexanol, benzylalcohol, 2,2,2-trifluoroethanol, 2,2,2-trichloroethanol, trichloromethanol, 1,1,1,3,3,3-hexafluoroisopropanol, nonafluoro t-butanol, phenol, fluoro- and chlorophenols and polysubstituted halogenated phenols such as o-chlorophenol, p-chlorophenol, o-fluorophenol, p-fluorophenol, pentafluorophenol and the like, 2-ethoxy ethanol, 2-methoxy-ethanol, 2-isopropoxy-ethanol, 1-methoxy-2-propanol, 1-ethoxycyclopropanol, 1,3-dimethoxy-2-propanol, 1,1-dimethoxy-ethanol, 2-methoxy-1-propanol, 2-methoxy-3-propanol and the like, dimethyloxime, N, N-dimethylethanolamine, hydroxylamines and mixtures thereof If the intent is to make aromatic non-distillable isocyanates the following alcohols are preferred: 2,2,2-trifluoroethanol, 2,2,2-trichloroethanol, trichloromethanol, 1,1,1,3,3,3-hexafluoroisopropanol, nonafluoro t-butanol, phenol, fluoro- and chlorophenols and polysubstituted halogenated phenols such as o-chlorophenol, p-chlorophenol, o-fluorophenol, p-fluorophenol, pentafluorophenol and the like, 2-ethoxy-ethanol, 2-methoxy-ethanol, 2-isopropoxy-ethanol, 1-methoxy-2-propanol, 1-ethoxycyclopropanol, 1,3-dimethoxy-2-propanol, 1,1-dimethoxy-ethanol, 2-methoxy-1-propanol, 2-methoxy-3-propanol and the like.

The required amount of alcohol depends of the exact nature of the amine and the reaction conditions and generally ranges between 1 and 100 moleq. alcohol/amine group. Even larger amounts are possible if the alcohol is used as a co-solvent.

The term "ionic liquid" as used herein, represents connections, which exhibit at least a cationic center and at least an anionic center, in particular those with at least one cation and at least one anion, more particularly those wherein the cation is organic.

Cations can be quatemairy ammoniums, phosphoniums, imidazoliums, H-pyrazoliums, pyridaziniums, pyrimidiniums, pyraziniums, pyrrolidiniums, guanidiniums, isouroniums, thiouroniums, five to at least six membered heterocyclic cations, which at least contain a phosphorous or a nitrogen atom as well as if necessary an oxygen or a sulfur atom. Examples are thiazolium, oxazolium, 1,2,4-triazolium or 1,2,3-triazolium, particularly preferred are five or six membered heterocycles, which contain two or three nitrogen atoms and a sulfur or an oxygen atom; 1,8-diazabicyclo[5.4.0]undec-7-enium-cation as well as 1,8-diazabicyclo[4.3.0]non-5-enium-cation and the like and mixed species with metal cations.

In principle, all anions are applicable. Anions can be halides and/or connections containing halogens, sulfates, sulfites, sulfonates, phosphates, phosphites, phosphonates, phosphinates, phosphonites, phosphinites, phosphonium amino acids, carbonic acids, borates, boronates, carbonates and carbonic acid esters, silicates and silicic acid acid esters, alkyl and/or aryl silane salts, carbonic acid imides, bis(sulphonyl)imides and sulphonamides, amides, nitrates, thiocyanates, alkoxides and aryloxides as well as complex metal ions.

Examples of suitable ionic liquids are 1-butyl-3-methyl imidazolium chloride, 1-butyl-3-methyl imidazolium bromide, 1-butyl-3-methyl imidazolium hexafluorophosphate, 1-butyl-3-methyl imidazolium iodide, 1-butyl-3-methyl imidazolium methylsulfate, 1-butyl-3-methyl imidazolium octylsulfate, 1-butyl-3-methyl imidazolium tetrafluoroborate, 1-butyl-3-methyl imidazolium trifluoromethanesulfonate, 1-butyl-3-methyl imidazolium methane sulfonate, 1-butyl-3-methyl imidazolium trifluoroacetate, 1-butyl-3-methyl imidazolium dicyanamide, 1-butyl-3-methyl imidazolium acetate, 1-butyl-3-methyl imidazolium hexafluoroantimonate, 1-butyl-3-methyl imidazolium hydrogen sulfate, 1-butyl-3-methyl imidazolium 2-(2-methoxyethoxy)ethyl sulfate, 1-butyl-3-methyl imidazolium nitrate, 1-butyl-3-methyl imidazolium tetrachloroaluminate, 1-butyl-3-methyl imidazolium thiocyanate, 1,3-dimethylimidazolium trifluoromethanesulfonate, 1-ethyl-3-methylimidazolium bis[oxalato]borate, 1-ethyl-3-methylimidazolium bromide, 1-ethyl-3-methylimidazolium chloride, 1-ethyl-3-methylimidazolium hexafluorophosphate, 1-ethyl-3-methylimidazolium methylsulfate, 1-ethyl-3-methylimidazolium p-toluenesulfonate, 1-ethyl-3-methylimidazolium tetrafluoroborate, 1-ethyl-3-methylimidazolium thiocyanate, 1-ethyl-3-methylimidazolium trifluoromethanesulfonate, 1-ethyl-3-methylimidazolium trifluoroacetate, 1-ethyl-3-methylimidazolium bis(pentafluoroethyl)phosphinate, 1-hexadecyl-3-methylimidazolium chloride, 1-hexyl-3-methylimidazolium bis(trifluoromethylsulfonyl)imide, 1-hexyl-3-methylimidazolium chloride, 1-hexyl-3-methylimidazolium hexafluorophosphate, 1-hexyl-3-methylimidazolium tetrafluoroborate, 1-hexyl-3-methylimidazolium tris(pentafluoroethyl)trifluorophosphate, 3-methyl-1-octadecylimidazolium hexafluorophosphate, 3-methyl-1-octadecylimidazolium, 3-methyl-1-octadecylimidazolium tris(pentafluoroethyl)trifluorophosphate, 3-methyl-1-octylimidazolium bis(trifluoromethylsulfonyl)imide, 3-methyl-1-octylimidazolium chloride, 3-methyl-1-octylimidazolium hexafluorophosphate, 3-methyl-1-octylimidazolium octylsulfate, 3-methyl-1-octylimidazolium tetrafluoroborate, 3-methyl-1-tetradecylimidazolium tetrafluoroborate, 1-propyl-3-methylimidazolium iodide, 1-butyl-2,3-dimethylimidazolium chloride, 1-butyl-2,3-dimethylimidazolium hexafluorophosphate, 1-butyl-2,3-dimethylimidazolium iodide, 1-butyl-2,3-dimethylimidazolium octylsulfate, 1-butyl-2,3-dimethylimidazolium tetrafluoroborate, 1-ethyl-2,3-dimethylimidazolium bromide, 1-ethyl-2,3-dimethylimidazolium chloride, 1-ethyl-2,3-dimethylimidazolium hexafluorophosphate, 1-ethyl-2,3-dimethylimidazolium p-toluenesulfonate, 1-ethyl-2,3-dimethylimidazolium tetrafluoroborate, 1-hexadecyl-2,3-dimethylimidazolium chloride, 1-hexyl-2,3-dimethylimidazolium chloride, 1,2,3-trimethylimidazolium iodide, 1,2,3-trimethylimidazoliumchloride, 1,2-dimethylimidazoliumchloride, 1-butylimidazoliumchioride, 1-methylimidazoliumchloride, 1-butyl-4-methylpyridiniumchloride, 1-butylpyridiniumchloride, methyltrioctylammoniumchloride, octyltrimethylammoniumchloride, 1,2,3-trimethylimidazoliumtetrachloroaluminate, 1,2-dimethylimidazoliumtetrachloroaluminate, 1,1-butyl-S-dimethylimidazoliumtetrachloroaluminate, butyl-3-methylimidazoliumtetrachloroaluminate, 1-ethyl-2,3-dimethylimidazoliumtetrachloroaluminate, 1,1-ethyl-S-methylimidazoliumtetrachloroaluminate, butyl-4-methylpyridiniumtetrachloroaluminate, N-butylpyridiniumtetrachloroaluminate, methyltrioctylammoniumtetrachloroaluminate, octyltrimethylammoniumtetrachloroaluminate, 1,2,3-trimethylimidazoliumethylsulfate, 1,2-dimethylimidazoliumethylsulfate, 1-butyl-2,3-dimethylimidazoliumethylsulfate, 1-butyl-3-methylimidazoliumethylsulfate, 1-ethyl-2,3-dimethylimidazoliumethylsulfate, 1-ethyl-3-methylimidazoliumethylsulfate, 1-butyl-4-methylpyridiniumethylsulfate, 1-butylpyridiniumethylsulfate, methyltrioctylammoniumethylsulfate, octyltrimethylammoniumethylsulfate, 1,2,3-trimethylimidazoliummethylsulfate, 1,2-dimethylimidazoliummethylsulfate, 1-butyl-2,3-dimethylimidazoliummethylsulfate, 1-ethyl-2,3-dimethylimidazoliummethylsulfate, 1-butyl-4-methylpyridiniummethylsulfate, 1-butylpyridiniummethylsulfate, methyltrioctylammoniummethylsulfate, octyltrimethylammoniummethylsulfate, 1,2,3-trimethylimidazoliummethylsulfonate, 1,2-dimethylimidazoliummethylsulfonate, 1-butyl-2,3-dimethylimidazoliummethylsulfonate, 1-butyl-3-methylimidazoliummethylsulfonate, 1-ethyl-2,3-dimethylimidazoliummethylsulfonate, 1-ethyl-3-methylimidazoliummethylsulfonate, 1-butyl-4-methylpyridiniummethylsulfonate, 1-butylpyridiniummethylsulfonate, methyltrioctylammoniummethylsulfonate, octyltrimethylammoniummethylsulfonate, 1,2,3-trimethylimidazoliumsulfate, 1,2-dimethylimidazoliumsulfate, 1-butyl-2,3-dimethylimidazoliumsulfate, 1-butyl-3-methylimidazoliumsulfate, 1-ethyl-2,3-dimethylimidazoliumsulfate, 1-ethyl-3-methylimidazoliumsulfate, 1-butyl-4-methylpyridiniumsulfate, 1-butylpyridiniumsulfate, methyltrioctylammoniumsulfate, octyltrimethylammoniumsulfate, 1,2,3-trimethylimidazoliumtosylate, 1,2-dimethylimidazoliumtosylate, 1-butyl-2,3-dimethylimidazoliumtosylate, 1-butyl-3-methylimidazoliumtosylate, 1-ethyl-2,3-dimethylimidazoliumtosylate, 1-ethyl-3-methylimidazoliumtosylate, 1-butyl-4-methylpyridiniumtosylate, 1-butylpyridiniumtosylate, methyltrioctylammoniumtosylate, octyltrimethylammoniumtosylate, N-hexylpyridinium bis(trifluoromethylsulfonyl)imide, N-butyl-3,4-dimethylpyridinium chloride, N-butyl-3,5-dimethylpyridinium chloride, N-butyl-3-methylpyridinium chloride, N-butyl-4-methylpyridinium bromide, N-butyl-4-methylpyridinium chloride, N-butyl-4-methylpyridiniumhexafluorophosphate, N-butyl-4-methylpyridinium tetrafluoroborate, N-butylpyridinium chloride, N-butylpyridinium hexafluorophosphate, N-butylpyridinium tetrafluoroborate, N-butylpyridinium trifluoromethanesulfonate, N-ethylpyridinium bromide, N-ethylpyridinium chloride, N-hexylpyridinium hexafluorophosphate, N-hexylpyridinium tetrafluoroborate, N-hexylpyridinium trifluoromethanesulfonate, N-octylpyridinium chloride, 1,1-dimethylpyrrolidinium iodide, 1-butyl-1-methylpyrrolidinium bis(trifluoromethylsulfonyl)imide, 1-butyl-1-methylpyrrolidinium chloride, 1-butyl-1-methylpyrrolidinium hexafluorophosphate, 1-butyl-1-methylpyrrolidinium tetrafluoroborate, 1-butyl-1-methylpyrrolidinium trifluoroacetate, 1-butyl-1-methylpyrrolidinium trifluoromethanesulfonate, 1-butyl-1-methylpyrrolidinium tris(pentafluoroethyl)trifluorophosphate, 1-butyl-1-methylpyrrolidinium bis[oxalato(2-)]borate, 1-hexyl-1-methylpyrrolidinium chloride, 1-methyl-1-octylpyrrolidinium chloride, trihexyl(tetradecyl)phosphonium bis(trifluoromethylsulfonyl)imide, trihexyl(tetradecyl)phosphonium bis[oxalato(2-)]-borate, trihexyl(tetradecyl)phosphonium chloride, trihexyl(tetradecyl)phosphonium hexafluorophosphate, trihexyl(tetradecyl)phosphonium tetrafluoroborate, trihexyl(tetradecyl)phosphonium tris(pentafluoroethyl)trifluorophosphate, methyltrioctylammonium bis(trifluoromethylsulfonyl)imide, methyltrioctylammonium trifluoroacetate, methyltrioctylammonium trifluoromethanesulfonate, ethyl-dimethyl-propylammonium bis(trifluoromethylsulfonyl)imide, guanidinium trifluoromethanesulfonate, guanidinium tris(pentafluoroethyl)trifluorophosphate, N"-ethyl-N,N,N',N'-tetramethylguanidinium trifluoromethanesulfonate, N"-ethyl-N,N,N',N'-tetramethylguanidinium tris(pentafluoroethyl)trifluorophosphate, O-ethyl-N,N,N',N'-tetramethylisouronium trifluoromethanesulfonate, O-ethyl-N,N,N',N'-tetramethylisouronium tris(pentaffuoroethyl)trifluorophosphate, S-ethyl-N,N,N',N'-tetramethylisothiouronium trifluoromethanesulfonate, S-ethyl-N,N,N',N'-tetramethylisothiouronium tris(pentafluoroethyl)trifluorophosphate.

Any type of organic or inorganic base with a base strength sufficient to remove a proton from the amine can be used. Required base strength is therefore dependent on the nature of the amine. The amount of base generally ranges between 0 and 3 moleq/amine group.

Examples of phosphazene compounds which can be employed as base in the process of the invention include, but are not limited to, imino-tris(dimethylamino)phosphorane, t-butyliminotris(dimethylamino)-phosphorane (P₁ -tBu), 1-t-butyl-4,4,4-tris(dimethylamino)-2,2-bis-[tris(dimethylamino)phosphoranylideneamino]-2.lambda.,4.lambda.-catenadi (phosphazene) (P₄-tBu), 2-t-butylimino-2-diethylamino-1,3-dimethyl-perhydro-1,3,2-diazaphosphorane (BEMP), t-butyliminotris(diethylamino) phosphorane, 2-t-octylimino-2-diethylamino-1,3-dimethyl-perhydro-1,3,2-diazaphosphorane, tetrakis[tris(dimethylamino)phosphoranylidene amino] phosphonium chloride (P₅) and the like, and mixtures of any two or more thereof.

Examples of organic, nitrogenous bases which can be employed in the process of the invention are selected from the group consisting of guanidine compounds, amidine compounds, O-alkyl urea, tertiary amines, aromatic amines, proton sponges and mixtures thereof including triethylamine, diethyl isopropylamine, trimethylamine, imidazole, amino benzimidazole, dimethyl amino pyridine, 1,8-bis(dimethylamino)naphthalene, adamanzane, Verkade's superbase, 1,2,4-triazolo[1,5-a]pyrimidine, H-1,2,3,-triazolo(4, 5-B)pyridine, 1,3,5-triazine, phthalocyanine, tetramethyl guanidine (TMG), cyclohexyltetramethyl guanidine (CyTMG), butyltetraethyl guanidine (n-BTEG), cyclohexyltetraethyl guanidine (CyTEG), tetraethyl guanidine (TEG), t-butyl-tetraethyl guanidine (t-BTEG), 7-methyl-1,5,7-triazabicyclo[4.4.0]dec-5-ene (MTBD), t-butyl-dimethyl formamidine (t-BDMF), t-butyldimethyl acetamidine (t-BDMA), 1,5-diazabicyclo[4.3.0]non-5-ene (DBN), 1,8-diazabicyclo[5.4.0] undec-7-ene (DBU) and the like, and mixtures of any two or more thereof.

Other examples of suitable base compounds include alkali metal hydroxides, alkaline earth metal hydroxides, alkaline earth metal oxides, alkali metal carbonates and alkaline earth metal carbonates such as sodium hydroxide, potassium hydroxide, lithium hydroxide, calcium hydroxide, magnesium hydroxide, calcium oxide, magnesium oxide, sodium carbonate, potassium carbonate, calcium carbonate, magnesium carbonate, and the like, and mixtures thereof

One can also use any type of the above homogeneous bases anchored or grafted on an organic or inorganic support such as:
- organic supports: ion exchange resins (Dowex and Amberlite type) and spacer modified polymer supports
- inorganic supports : ALPO (aluminiumphosphate), ALPON (aluminophosphate oxynitride, Al₂O₃ (Alumina), SiO₂ (silica, zeolites), carbon, TiO₂, V₂O₅, SiC and the like.

Further there can be used base in a crosslinked polymer form such as polyvinylpyiridines, aminobenzimidazole polymer or basic solids such as metal oxides, e.g. MgO, CaO, ZrO₂, Al₂O₃, hydrotalcites, clays and similar layered solids, SiO₂, TiO₂, MgAl₂O₄, mixed oxides such as Mg-Li-O, Li-La-O, Cs-La-O.

A different approach is to put the ionic liquid on a carrier known as Supported Ionic Liquid Phase Catalysis. This can be done in combination with a solid base or the carrier for the ionic liquid can contain base sites.

Next to the ionic liquid a co-solvent may be employed. This can simply be the alcohol which is already needed to make the carbamate and which is already exemplified above.

Other co-solvents can be aromatic hydrocarbons such as benzene, halogenated aromatic hydrocarbons such as monochlorobenzene, o-dichlorobenzene, trichlorobenzene or 1-chloro naphthalene, alkylated aromatic hydrocarbons like toluene, xylene, ethylbenzene, cumene or tetrahydronaphthalene, other functionalised aromatic hydrocarbons such as anisole, diphenylether, ethoxybenzene, benzonitrile, 2-fluoroanisole, 2,3-dimethylanisole, trifluorotoluene.

Co-solvents may also be alkanes such as n-pentane, n-hexane, n-heptane or higher branched alkanes, cyclic alkanes like cyclopentane, cyclohexane or derivatives thereof, halogenated alkanes like chloroform, dichloromethane, carbon tetrachloride and alkanes with other functional groups like diethylether, acetonitrile, propionitrile, dioxane and the like, ketones such as acetone and methyl ethyl ketone, amides such as N,N'-dimethyl formamide and N, N'-dimethylacetamide and esters such as ethylacetate and ethylbenzoate, N-methyl-2-pyrrolidone, dimethyl sulfoxide, sulfolane, pyridine and the like.

As solvents such connections can be used, that the amines are converted to salt melts in the presence of CO₂ and/or optionally a base.

Addition of CO₂ can be done in the gas phase by simply bubbling it through the reaction mixture or by high pressure injection in the gas phase or liquid phase or supercritical phase.

Reaction conditions range from the melting point of the ionic liquid until the decomposition temperature of the carbamate in the presence of alcohol (up to 250°C). Pressure ranges from atmospheric (bubbling CO₂) over supercritical conditions (> 73 bar) of several hundreds and thousands of bar CO₂ pressure. Preferred conditions are below 500 bar.

Product separation can be achieved by any known standard separation technique, which will depend of the exact nature of the product composition. Examples are distillation, extraction, filtration, adsorption and the like.

Decomposition of carbamates into isocyanates is a known process and has been described in the gas phase (US 3870739), liquid phase solvent free (US 5453536), liquid phase with a solvent (EP 611243), catalysed (US 4487713), stripping to promote distillation (EP 611243), continuous (US 5284969) and the like.

Distillable isocyanates can be removed from the reaction mixture directly by in situ thermolysis of the carbamate into the isocyanate and alcohol and by distillation of the isocyanate and/or the alcohol.

The overall process can be operated as a batch, continuous or semi-continuous process. Other additional benefits arising from various embodiments of the present invention include :
(a) economic attractiveness from the use of CO₂ together with minimisation of the amount of effluent,
(b) economic attractiveness from the use of a single step production from amines to carbamates and potentially production of isocyanates during work-up of the reaction mixture,
(c) production of isocyanates with low levels of so-called hydrolysable chlorine impurities.

## Claims

1. Method for the synthesis of carbamates comprising the step of reacting an amine compound, an alcohol and CO₂ in the presence of an ionic liquid and optionally in the presence of a base.

2. Method according to claim 1 wherein the amine compound is selected from the group consisting of 1,6 hexylene diamine, 1,4-cyclohexane diamine, 4,4'-dicyclohexylmethane diamine, metaxylene diamine, isophorone diamine, tetramethyl metaxylene diamine, 1,3-bis(aminomethyl)cyclohexane, isomers of methylene diphenylene diamine and mixtures of various isomers and homologues of aniline-formaldehyde condensates and isomers of tolulene diamine.

3. Method according to claim 1 or 2 wherein the alcohol is selected from the group consisting of 2,2,2-trifluoroethanol, 2,2,2-trichloroethanol, trichloromethanol, 1,1,1,3,3,3-hexafluoroisopropanol, nonafluoro t-butanol, phenol, fluoro- and chlorophenols and polysubstituted halogenated phenols such as o-chlorophenol, p-chlorophenol, o-fluorophenol, p-fluorophenol, pentafluorophenol and the like, 2-ethoxy-ethanol, 2-methoxy-ethanol, 2-isopropoxy-ethanol, 1-methoxy-2-propanol, 1-ethoxycyclopropanol, 1,3-dimethoxy-2-propanol, 1,1-dimethoxy-ethanol, 2-methoxy-1-propanol and 2-methoxy-3-propanol.

4. Method according to any one of claims 1 to 3 wherein the amount of alcohol ranges from 1 to 100 mole equivalents of alcohol per amine group.

5. Method according to any one of the preceding claims wherein the amount of base ranges from 0 to 3 mole equivalents of base per amine group.

6. Method according to any one of the preceding claims wherein the reaction is carried out in the presence of a co-solvent.

7. Method according to any one of the preceding claims wherein the carbamate is further decomposed into isocyanate and alcohol.
